(19) Europäisches Patentamt / European Patent Office / Office européen des brevets

(11) **EP 4 032 542 A1**

(12) # DEMANDE DE BREVET EUROPEEN

(43) Date de publication:
**27.07.2022 Bulletin 2022/30**

(21) Numéro de dépôt: **22156631.8**

(22) Date de dépôt: **13.05.2015**

(51) Classification Internationale des Brevets (IPC):
*A61K 38/44* (2006.01)     *A61K 31/7004* (2006.01)
*A61K 33/30* (2006.01)     *A61K 35/644* (2015.01)
*A61P 17/02* (2006.01)     *A23L 21/25* (2016.01)
*A61K 9/00* (2006.01)     *A61K 9/06* (2006.01)
*A61P 17/00* (2006.01)     *A61P 29/00* (2006.01)
*A61P 31/02* (2006.01)     *A61P 31/04* (2006.01)
*A61K 31/702* (2006.01)

(52) Classification Coopérative des Brevets (CPC):
(C-Sets disponibles)
A61K 38/443; A23L 21/25; A61K 9/0014;
A61K 9/06; A61K 31/7004; A61K 31/702;
A61K 33/30; A61K 35/644; A61P 17/00;
A61P 17/02; A61P 29/00; A61P 31/02;
A61P 31/04; C12Y 101/03004     (Cont.)

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **14.05.2014 FR 1454293**

(62) Numéro(s) de document de la (des) demande(s) initiale(s) en application de l'article 76 CBE:
**15736533.9 / 3 142 687**

(71) Demandeur: **Melipharm**
**87000 Limoges (FR)**

(72) Inventeurs:
• **MALEPEYRE, Carmen**
**87430 VERNEUIL SUR VIENNE (FR)**

• **RAYNAUD, Anaïs**
**87410 LE PALAIS SUR VIENNE (FR)**
• **QUERO, Fabien**
**33110 LE BOUSCAT (FR)**

(74) Mandataire: **Aquinov**
**Allée de la Forestière**
**33750 Beychac et Caillau (FR)**

Remarques:
Cette demande a été déposée le 14-02-2021 comme demande divisionnaire de la demande mentionnée sous le code INID 62.

(54) **COMPOSITION ANTIMICROBIENNE**

(57) L'objet de l'invention est une composition comprenant un mélange constitué par au moins un glucide, de la glucose oxydase et de l'oxyde de zinc.

Cette composition antimicrobienne à application topique est particulièrement utile pour le soin et le traitement des affections de la peau et des muqueuses.

**EP 4 032 542 A1**

(52) Classification Coopérative des Brevets (CPC):
(Cont.)

C-Sets
**A61K 31/7004, A61K 2300/00;**
**A61K 31/702, A61K 2300/00;**
**A61K 33/30, A61K 2300/00;**
**A61K 35/644, A61K 2300/00;**
**A61K 38/443, A61K 2300/00**

**Description**

La présente invention concerne une composition comprenant un mélange d'au moins un glucide, de glucose oxydase et d'oxyde de zinc comme composition antimicrobienne, et son utilisation sur la peau ou les muqueuses.

Il est très souvent nécessaire d'avoir recours à des compositions antimicrobiennes en application topique, notamment pour le traitement des plaies, ou pour le traitement d'infections microbiennes cutanées ou de pathologies de la peau avec une origine microbienne.

Parmi les produits utilisés pour le traitement des plaies, on connaît notamment les crèmes, pommades ou pansements à l'argent, à l'iode ou des pansements à base de miel.

Les pansements à l'argent sont souvent très coûteux et présentent généralement une cytotoxicité envers les cellules du derme et de l'épiderme (Lansdown ABG. A pharmacological and toxicological profile of silver as an antimicrobial agent in medical devices. Adv Pharm Sci 2010; 2010:910686. Epub 2010 Aug 24, Burd A, Kwok CH, Hung SC, et al. A comparative study of the cytotoxicity of silver-based dressings in monolayer cell, tissue explant, and animal models. Wound Repair Regen 2007; 15(1): 94-104) et utilisés en pédiatrie, ils peuvent provoquer des élévations du taux sanguin d'argent.

Les pansements à l'iode sont également cytotoxiques et leur activité antimicrobienne diminue fortement en présence de matière organique (pus, fibrine ou nécrose). De plus, il existe des risques de sensibilisation avec eczéma de contact et allergie à ces produits à base d'iode. Depuis plusieurs années, le miel est devenu un produit d'intérêt dans le domaine médical pour le traitement de certaines infections au niveau des plaies, cependant il existe de grandes variabilités sur leur propriétés antimicrobiennes suivant leur origines. Toutefois, les miels possédant une activité antimicrobienne importante présentent souvent une cytotoxicité envers les cellules de la peau. Il est donc très difficile de trouver un miel à la fois très antimicrobien et non-cytotoxique.

Les demandes WO-2005/112852, WO-2007/045931, US2004/121020 ou WO-2004/000339 par exemple, décrivent des compositions mielleuses, des pansements ou dérivés de pansements à base de miel de manuka associé à des agents texturants ou à d'autres principes actifs. Les compositions décrites permettent de décontaminer les plaies et favorisent ainsi leur cicatrisation.

Toutefois, ces compositions ne sont pas nécessairement satisfaisantes en termes d'efficacité, en particulier sur la migration et la prolifération cellulaire, nécessaires à la cicatrisation.

De plus, la fabrication de ces compositions, est souvent complexe et couteuse, en particulier du fait de l'utilisation de procédés de fabrication complexes (association à des interfaces) et/ou de l'ajout de principes actifs à des miels spécifiques et coûteux (comme par exemple le miel de manuka).

Dans la demande FR2995532 il est décrit des compositions à base de miel qui pallient ces inconvénients et qui outre leur efficacité antibactérienne sont capables d'inhiber la formation de biofilms, de présenter un effet bactéricide vis-à-vis de biofilms déjà formés, et d'empêcher l'adhésion des bactéries.

L'objectif de la présente invention est de proposer une composition antimicrobienne différente au moins aussi efficace que celles décrites dans la demande FR29995532.

A cet effet, l'invention vise une composition comprenant un mélange constitué par au moins un glucide, de glucose oxydase et de l'oxyde de zinc, notamment pour son utilisation comme produit antimicrobien à application topique.

Le mélange selon l'invention présente un effet synergique important et surprenant en regard des compositions existantes, qui permet de proposer une composition présentant une activité antimicrobienne qui dépasse celle des produits à base de miel existant actuellement, sans variation de cytotoxicité.

D'autres caractéristiques et avantages ressortiront de la description en détails de l'invention qui va suivre.

L'invention vise donc une composition comprenant un mélange constitué par au moins un glucide, de glucose oxydase et d'oxyde de zinc.

Le glucide est très préférentiellement au moins le glucose. Préférentiellement il s'agit du glucose seul ou d'un mélange de glucides contenant notamment du glucose.

La source de glucide peut être notamment du miel. Le miel peut être du miel d'origine naturelle ou du miel artificiel ou un mélange de plusieurs miels naturel(s) et/ou artificiel(s).

Par miel artificiel on entend au moins un sucre. Il peut s'agir d'une combinaison d'au moins deux sucres.

Dans le cas où la source de glucide est du miel naturel il peut s'agir préférentiellement du miel de thym et/ou de miellat et/ou de sarrasin et/ou de manuka et/ou leurs mélanges. Il peut s'agir par exemple d'un miel tel que décrit dans la demande FR1258722.

Dans le cas où la source de glucide est du miel artificiel, il est préférentiellement constitué majoritairement de glucose et/ou fructose et/ou saccharose.

Selon un mode de réalisation particulièrement adapté, le glucide est le glucose.

Le ou les glucides représentent préférentiellement 1% à 99% en poids du poids total de la composition, encore plus préférentiellement entre 1 et 60%.

La composition selon l'invention comprend en plus du ou des glucides, de la glucose oxydase et de l'oxyde de zinc.

La glucose oxydase est une enzyme qui permet de catalyser la réaction d'oxydation du glucose en peroxyde d'hydrogène en présence d'eau. Cette enzyme peut être d'origine naturelle ou biotechnologique. Elle est principalement présente dans les sources microbiennes et obtenue par fermentation de champignons comme *l'Aspergillus niger.*

La glucose oxydase est présente à une dose d'au moins 0,025 mU (mili-unité) par gramme de composition selon l'invention, préférentiellement entre 0,5 mU et 50 U par gramme de composition.

Si le glucide est du miel, lequel peut produire du peroxyde d'hydrogène grâce à la glucose oxydase qu'il contient naturellement, la quantité de glucose oxydase qu'il contient ne sera pas suffisante pour obtenir l'activité et l'efficacité liées à la présence de glucose oxydase telle que définie dans l'invention. La supplémentation du miel et autres constituants avec de la glucose oxydase à une dose d'au moins 0,025 mU est nécessaire pour obtenir les résultats décrits dans l'invention. Dans le cas où la composition contient du miel, la composition est bien supplémentée en glucose oxydase, en plus de celle pouvant éventuellement être contenue dans le miel et cette dose supplémentaire est préférentiellement d'au moins 0,025mU. L'oxyde de zinc est un composé minéral de formule ZnO utilisé dans de nombreuses applications, notamment dans les préparations topiques. Pour la présente invention, la taille des particules d'oxyde de zinc utilisée est très préférentiellement inférieure à 50 $\mu$m. L'oxyde de zinc représente préférentiellement entre 0,1% et 40% en poids du poids total de la composition encore plus préférentiellement entre 0,1% et 10%.

La composition selon l'invention peut être composée exclusivement de glucide(s), de glucose oxydase et d'oxyde de zinc ou bien contenir d'autres constituants.

Elle peut notamment comprendre également en plus du mélange, un ou plusieurs constituants choisi(s) parmi, la vanilline, le caprylyl glycol, le pentylène glycol, le 1,2-hexanediol, un extrait de propolis, le methylglyoxal, le glycérol, le propylène glycol, le polyéthylène glycol, la pectine, les gommes, le carraghénane, la xanthane, les sels d'alginate, la cellulose et ses dérivés, l'amidon et ses dérivés, le chitosan, les triglycérides, les vitamines, les beurres, les cires, le glycéryl stéarate, la vaseline, les paraffines, le kaolin, la bentonite, la lanoline.

Elle peut aussi contenir des excipients choisis parmi les excipients dermatologiquement compatibles et/ou applicables sur la peau et les muqueuses afin d'obtenir une composition sous forme de poudre, ou sous forme liquide telle qu'une lotion, ou semi-solide tels qu'une poudre à usage cutané, un spray, une crème, une pommade, un gel, une pâte, une émulsion, un suppositoire, un ovule. Il peut s'agir par exemple d'excipients tels que des sucres et dérivés de sucres, des polysaccharides (pectines, amidon et dérivés, alginates et dérivés, chitosan et dérivés, dérivés de cellulose, des gommes, des béta-glucanes), des polymères de synthèse, des cires, des huiles naturelles ou non, des beurres, des produits minéraux (silice, talc, argiles, oxyde de titane), des glycérides et autres esters gras, des tensio-actifs, l'eau, l'éthanol, le propylène glycol, le butylène glycol, le glycérol, le sorbitol, des composés de la matrice extracellulaire comme de l'acide hyaluronique ou du collagène, des hydrocarbures et silicones, des protéines et peptides et autres excipients connus de l'homme de métier.

La composition peut aussi contenir des additifs auxiliaires de formulation tels que des tensioactifs, des agents gélifiants, des agent absorbants, des agents humectants, des solvants, des agents d'étalement, des stabilisants, des complexants, des modificateurs rhéologiques, des conservateurs, des antioxydants et antimicrobiens, des colorants et des parfums.

La composition selon l'invention peut être stérile, c'est-à-dire qu'elle a subit un procédé de stérilisation. La stérilisation est préférentiellement réalisée par rayonnement gamma.

La composition selon l'invention peut être obtenue par simple mélange des constituants. Préférentiellement, elle est obtenue par un mélange homogène de poudre ou par dispersion des poudres dans une composition de miel.

La composition selon l'invention peut se présenter sous différentes formes poudres ou liquides ou semi-liquides, notamment poudre à usage cutané, spray, lotion, crème, émulsion, gel, pommade, ovule, suppositoire.

La composition est utilisée comme produit antimicrobien à application topique, en particulier comme produit de santé ou composition cosmétique. En effet, le mélange selon l'invention, agit en synergie pour à la fois :

- limiter la prolifération microbienne : la composition est capable de détruire les germes sur la peau, en particulier au niveau des plaies,
- inhiber la formation des biofilms sur la peau et/ou détruire des biofilms déjà formés sur la peau, en particulier au niveau des plaies,
- prévenir ou lutter contre l'adhésion des micro-organismes sur la peau, en particulier au niveau des plaies.

La diminution de la charge microbienne permet de favoriser indirectement la cicatrisation. De plus, La composition selon l'invention peut également permettre de favoriser la prolifération des fibroblastes pour un effet cicatrisant.

Les effets obtenus avec le mélange des trois constituants de la composition selon l'invention sont bien meilleurs que ceux obtenus avec chacun des constituants seuls ou des mélanges de chacun des constituants deux par deux. Il existe un effet synergique surprenant.

Au niveau microbien, chacun des trois constituants de la composition selon l'invention peut permettre de limiter très faiblement la prolifération microbienne, mais lorsqu'ils sont utilisés ensemble, la diminution de la prolifération microbienne est plus importante que la somme des limitations induites par les constituants seuls et il existe un effet synergique surprenant. Selon l'invention, la composition peut donc être avantageusement utilisée comme produit de santé à application topique sur la peau ou les muqueuses chez l'homme ou l'animal, très préférentiellement comme :

- produit de santé, en particulier dispositif médical à des fins thérapeutiques destiné à être appliqué sur des irritation ou des lésions cutanées notamment pour :

  • le traitement des brûlures de premier et second degré,
  • le traitement et/ou la prévention des désunions post opératoires de cicatrice,
  • le traitement et/ou la prévention post-opératoire des cavités résiduelles des sinus pilonidaux,
  • le traitement des cicatrices chirurgicales infectées après mise à plat,
  • le traitement des ulcères et escarres en phase de bourgeonnement,
  • le traitement des dermabrasions, plaies traumatiques et/ou chirurgicales,
  • le traitement des plaies aigües et chroniques,
  • le traitement des plaies cancérologiques,
  • le traitement des emplacements de stomies,
  • le traitement des plaies superficielles.

- composition destinée au traitement des irritations et/ou infections d'origine microbienne, en particulier les lésons de grattage, les érythèmes fessiers, l'acné, les dermatites, l'impétigo, les furoncles, les folliculites, les panaris et les mycoses, notamment comme composition cosmétique,
- gel buccal, notamment destiné à traiter les aphtes et les mucites,
- stick ou baume à lèvre destiné à traiter les gerçures,
- crème pour les mains et les pieds destinée notamment au traitement des crevasses et des engelures,
- baume destiné à la prévention, au soin et/ou au traitement des crevasses du mamelon,
- gel vaginal pour traiter des lésions cutanées et les infections microbiennes,
- suppositoire ou crème rectale pour traiter des lésions et irritations rectales.

Par dispositif médical à des fins thérapeutiques on entend un produit de santé destiné à être utilisé chez l'homme ou l'animal à des fins notamment de prévention, de contrôle, de traitement et/ou d'atténuation d'une maladie, d'une blessure, d'une affection ou d'une irritation cutanée.

S'il s'agit d'un produit de santé pour la cicatrisation de lésions cutanées se présentant sous forme de gel, crème ou pommade, la composition selon l'invention peut en particulier s'appliquer selon le protocole d'utilisation suivant :

- rincer la plaie et sécher doucement,
- recouvrir l'intégralité de la plaie ou le pansement secondaire d'une pellicule de la composition selon l'invention,
- recouvrir de compresses et d'un pansement occlusif durant les premières applications pour les plaies très exsudatives.

En phase de détersion, et en fonction de l'état de la plaie, il est conseillé de refaire le pansement une à deux fois par 24 heures.

En phase de bourgeonnement, il est conseillé de refaire le pansement toutes les 48 à 72 heures.

En phase d'épithélialisation, il est conseillé de refaire le pansement tous les 3 ou 4 jours. L'invention est à présent illustrée par des exemples de composition et des résultats d'essais démontrant son efficacité.

## EXEMPLES DE COMPOSITION SELON L'INVENTION

### Exemple 1

La composition de l'exemple 1 est une poudre constituée de :

- 98,9% Glucose
- 1% ZnO
- 0,1% Glucose oxydase

La composition est obtenue par mélange des composés.

### Exemple 2

La composition de l'exemple 2 est une pommade constituée de :

- qsp 100% mélange de miels naturels
- 25% de triglycérides
- 11% de cire d'abeille blanche
- 7,5% de beurre de karité
- 1,83% de tocopherol acétate
- 5% de glyceryl mono stearate
- 5% ZnO
- 0,01% Glucose oxydase

les pourcentages étant des pourcentages en poids par rapport au poids total de la matière sèche de la composition.

La composition est obtenue par la mise en œuvre des étapes suivantes :

- disperser finement les poudres dans le miel,
- chauffer le mélange à moins de 40°C,
- chauffer la phase lipophile (cire, beurre et glycérides) à environ 70°C pour faire fondre les solides,
- quand la phase lipophile a atteint la température de 40°C, ajouter le tocophérol acétate et le mélange de miels et de poudres,
- mélanger la pommade jusqu'à refroidissement.

### Exemple 3

La composition de l'exemple 3 est une pâte constituée de :

- qsp 100% miel artificiel
- 2% ZnO
- 0,1% Glucose oxydase

La composition est obtenue par mélanges des composés.

### Exemple 4

La composition de l'exemple 4 est une pâte constituée de :

- qsp 100% miel de sarrasin
- 1% ZnO
- Glucose oxydase 0,1%

La composition est obtenue par mélanges des composés à température ambiante.

### Exemple 5

La composition de l'exemple 5 est un gel constitué de :

- qsp 100% miel de miellat
- Glycérol 15%
- PEG 1500 17,5%
- Pectine 4%
- ZnO 10%
- Glucose oxydase 1,5%

La composition est obtenue par :

- mélange des poudres, puis à 40°C ajout du Glycérol et du miel,
- dissolution des PEG à 70°C,

6

- refroidissement à 40°C,
- ajout de la base miel dans les PEG à 40°C, mélange jusqu'à refroidissement.

## EVALUATION DE L'EFFICACITE DE LA COMPOSITION SELON L'INVENTION

Des essais ont été réalisés pour démontrer l'efficacité antimicrobienne de plusieurs compositions selon l'invention d'une part et comparer cette efficacité à celle du constituants seuls.

### 1. Efficacité antibactérienne et anti-biofilm in vitro

Pour la réalisation des tests d'efficacité, plusieurs formules ont été testés :

- formule 1: Miel seul
- formule 2: Miel + 0,1% de glucose oxydase
- formule 3: ZnO seul 1%
- formule 4: Miel + ZnO 1%
- formule 5: Miel + 0,1% de glucose oxydase + 1% de ZnO (non irradiée)
- formule 6: Miel + 0,1% de glucose oxydase + 1% de ZnO + excipients (formule en vieillissement normal = 4 mois; irradiée)
- formule 7: Miel + 0,1% de glucose oxydase + 1% de ZnO + excipients (formule en vieillissement accéléré 92 jours = 1 an ; irradiée)
- formule 8: glucose oxydase 0,1%
- formule 9: glucose seul
- formule 10: glucose + glucose oxydase 0,1%
- formule 11: glucose + ZnO 1%
- formule 12: glucose + glucose oxydase 0,1% + ZnO 1%

Les souches microbiennes utilisées pour les essais sont les bactéries :

- *P. aeruginosa*
- *S. aureus*

Les essais sont réalisés sur microplaques pour évaluation de la CMI (Concentration Minimale Inhibitrice).

### a. Tests de prolifération bactérienne

La prolifération bactérienne en présence des produits à tester est déterminée par une méthode en microplaque 96 puits. Chaque puits est inoculé avec 50 $\mu$L de suspension bactérienne à tester réalisée dans un bouillon Muller-Hinton (MH) + 150 $\mu$L de solution de produit à tester dilué. La concentration bactérienne dans le puits est fixée à $10^6$ CFU/mL.
Le témoin positif (50 $\mu$L de suspension bactérienne + 150 $\mu$L de milieu MH) correspond au 100% de prolifération bactérienne. Le témoin négatif (200 $\mu$L de milieu de culture) correspond au 0% de prolifération bactérienne.
Chaque échantillon est testé en triplicata. Les densités optiques (DO) sont lues une première fois au temps 0 (T0) à 450 nm. Les plaques sont ensuite incubées 24h à 37°C sous agitation. Au terme de l'incubation la DO est remesurée à 450 nm (T24). Le pourcentage de prolifération est calculé ainsi :

$$\text{Prolifération} = (DO_{T24} - DO_{T0}) / (DO_{T24 \text{ Témoin +}} - DO_{T0 \text{ Témoin +}})$$

Les résultats obtenus sont présentés dans les tableaux 1 et 2 ci-dessous :

*Tableau 1: Influence des actifs utilisés et des formules développées à partir de ces actifs sur la prolifération de P aeruginosa. Les formules sont testées en microplaques aux concentrations de 1,5% 3%, 6% et 9% (v/v). Les pourcentages de prolifération sont calculés à partir du témoin positif correspondant au milieu non traité (milieu de culture seul = 100% de prolifération)*

| Formules testées | 1.5% | 3% | 6% | 9% |
|---|---|---|---|---|
| Formule 1 | 92% | 98% | 48% | 6% |

(suite)

| Formules testées | 1.5% | 3% | 6% | 9% |
|---|---|---|---|---|
| Formule 2 | 76% | 86% | 40% | -7% |
| Formule 3 | 119% | 125% | 99% | 55% |
| Formule 4 | 107% | 107% | 39% | -4% |
| Formule 5 (invention) | 72% | 30% | -4% | -11% |
| Formule 6 (invention) | 64% | 44% | -31% | -30% |
| Formule 7 (invention) | 86% | 41% | -14% | -15% |
| Formule 8 | 96% | 96% | 91% | 86% |
| Formule 9 | 100% | 206% | 206% | 113% |
| Formule 10 | 96% | 98% | 89% | 79% |
| Formule 11 | 156% | 86% | 60% | 41% |
| Formule 12 (invention) | 94% | 63% | 31% | 0% |

On constate que les formules 5, 6, 7 et 12 entrainent une forte diminution de la prolifération de *P. aeruginosa*. Cette diminution est bien plus importante pour toutes les formules contenant au moins un glucide, la GOD et le ZnO par comparaison aux formules contenant soit au moins un glucide et GOD (formules 2 et 10) ou au moins un glucide et ZnO (formules 4 et 11). Pour la formule 12 les proliférations bactériennes diminuent respectivement de 79% et 41% à la concentration de 9% v/v pour atteindre la CMI. La CMI passe de 9% v/v à 6% v/v pour les formules où la source de glucide est du miel.

*Tableau 2: Influence des actifs utilisés et des formules développées à partir de ces actifs sur la prolifération de S. aureus. Les formules sont testées en microplaques aux concentrations de 1,5% 3%, 6% et 9% (v/v). Les pourcentages de prolifération sont calculés à partir du témoin positif correspondant au milieu non traité (milieu de culture seul = 100% de prolifération)*

| Formules testées | 1.5% | 3% | 6% | 9% |
|---|---|---|---|---|
| Formule 1 | 96% | 49% | -4% | -29% |
| Formule 2 | 75% | 37% | 7% | -34% |
| Formule 3 | 109% | 117% | 105% | 75% |
| Formule 4 | 90% | 17% | -13% | -44% |
| Formule 5 (invention) | -2% | 4% | 3% | -30% |
| Formule 6 (invention) | 4% | -17% | -10% | -58% |
| Formule 7 (invention) | 7% | -4% | -37% | -57% |
| Formule 8 | 81% | 72% | 54% | 46% |
| Formule 9 | 96% | 86% | 83% | 72% |
| Formule 10 | 92% | 95% | 72% | 65% |
| Formule 11 | 78% | 25% | 8% | -8% |
| Formule 12 (invention) | 52% | 2% | -23% | -53% |

On constate que les formules 5, 6, 7 et 12 entrainent une forte diminution de la prolifération de S. *aureus.* Cette diminution est bien plus importante pour toutes les formules contenant au moins un glucide, la GOD et le ZnO par comparaison aux formules contenant soit au moins un glucide et GOD (formules 2 et 10) ou au moins un glucide et ZnO (formules 4 et 11). Pour la formule 12 les proliférations bactériennes diminuent respectivement de 95% et 25% à la concentration de 3% v/v pour atteindre la CMI. La CMI passe de 9% v/v à 6% v/v pour les formules où la source de glucide est du miel.

b. Activité antibiofilm

L'activité antibiofilm en présence des produits à tester est déterminée par une méthode en microplaque 96 puits. Chaque puits est inoculé avec 50 µL de suspension bactérienne à tester réalisée dans un bouillon Tripticase soja +1% glucose (TS+1%Glu) + 150 µL de solution de produit à tester dilué. La concentration bactérienne dans le puits est fixée à $10^6$ CFU/mL. Le témoin positif (50 µL de suspension bactérienne + 150 µL de milieu TS+1%Glu) correspond au 100% de prolifération bactérienne. Le témoin négatif (200 µL de milieu de culture) correspond au 0% de prolifération bactérienne. Chaque échantillon est testé en triplicata. Les plaques sont ensuite incubées 24h à 37°C. Au terme de l'incubation le surnageant est éliminé, la microplaque est lavée 3 fois avec 200µL/puits d'eau distillée. La plaque est alors incubée à l'étuve à 60°C pendant 30 minutes. Une solution de cristal violet (CV) à 0,5% est ajoutée et la plaque est incubée 20 minutes à température ambiante. Au terme de l'incubation la solution de CV est retirée et la microplaque est lavée à nouveau 3 fois avec 200µL/puits d'eau distillée. La plaque est alors séchée une nuit à 60°C et enfin 100µL/puits d'une solution d'acide acétique à 33% sont ajoutés dans les puits. La plaque est alors photographiée. Les puits dans lesquels le biofilm s'est formé sont violet (+) alors que les puits dans lesquels il n'y a pas eu formation de biofilms sont clairs (-).

Les résultats obtenus sont présentés dans les tableaux 3 et 4 ci-dessous :

*Tableau 3: Influence des actifs utilisés et des formules développées à partir de ces actifs sur la prolifération de P aeruginosa. Les formules sont testées en microplaques aux concentrations de 1,5% 3%, 6% et 9% (v/v). Les biofilms sont révélés par une solution de cristal violet à 0,5%. Le témoin positif correspond au témoin de milieu de culture + P. aeruginosa, le témoin négatif correspond au milieu de culture sans bactéries*

| Formules testées | 1,5% | 3% | 6% | 9% |
|---|---|---|---|---|
| Formule 1 | + | + | + | - |
| Formule 2 | + | + | + | - |
| Formule 3 | + | + | + | + |
| Formule 4 | + | + | + | - |
| Formule 5 (invention) | + | + | - | - |
| Formule 6 (invention) | + | + | - | - |
| Formule 7 (invention) | + | + | - | - |

On constate une diminution de l'activité anti-biofilm à 6% v/v avec l'association {miel + glucose oxydase + ZnO} sur *P. aeruginosa*.

*Tableau 4: Influence des actifs utilisés et des formules développées à partir de ces actifs sur la prolifération de S. aureus. Les formules sont testées en microplaques aux concentrations de 1,5% 3%, 6% et 9% (v/v). Les biofilms sont révélés par une solution de cristal violet à 0,5%. Le témoin positif correspond au témoin de milieu de culture + S. aureus, le témoin négatif correspond au milieu de culture sans bactéries*

| Formules testées | 1,5% | 3% | 6% | 9% |
|---|---|---|---|---|
| Formule 1 | + | + | - | - |
| Formule 2 | + | + | - | - |
| Formule 3 | + | + | + | + |
| Formule 4 | + | + | - | - |
| Formule 5 (invention) | - | - | - | - |
| Formule 6 (invention) | - | - | - | - |
| Formule 7 (invention) | - | - | - | - |

On constate une diminution de l'activité antibiofilm à 1,5% avec l'association {miel + glucose oxydase + ZnO} sur *S. aureus*.

c. Démonstration de l'effet synergique

PRINCIPE DU TEST DE BERENBAUM

Le test de Berenbaum permet de déterminer des effets additifs, synergiques ou antagonistes entre deux produits mis en mélanges aux contacts de cellules (Berenbaum, 1977). Ce test fut développé afin de déterminer à l'aide de données expérimentales insérées dans une formule mathématique, l'impact de plusieurs composés mélangés. Il est applicable à de nombreux domaines de la biologie. Ce test est appliqué ici au {miel + GOD } et {ZnO} à partir des résultats de prolifération bactérienne obtenus sur les souches *S aureus* et *P aeruginosa.*

L'analyse des résultats de prolifération se fait sur la base d'une recherche d'activité équivalente pour chaque composé. Par exemple, l'activité recherchée est prolifération de 50%. La dose d'actif équivalente nécessaire pour obtenir 50% de prolifération (dans le cas de notre exemple) est désignée par $A_{éq}$ pour {miel + GOD }. $B_{éq}$ désigne la dose équivalente nécessaire pour obtenir le même effet (50% de prolifération) avec {ZnO}.

Dans un second temps, le mélange des deux composés permet de déterminer les doses de chaque produit à introduire ensemble pour obtenir le même effet (50% de prolifération) qu'avec $A_{éq}$ ou $B_{éq}$. On désigne alors la dose nécessaire de {miel + GOD } comme dose de A et celle de {ZnO} comme dose de B.

Par la suite, il est possible d'introduire ces données dans l'équation suivante :

$$Indice\ de\ Berenbaum = \frac{A}{A_{éq}} + \frac{B}{B_{éq}}$$

- Si le résultat de cette équation est égal à 1, l'effet des 2 composés sera additif
- Si le résultat est inférieur à 1, nous pourrons parler de synergie.
- En revanche si le résultat est supérieur à 1 les composés sont désignés comme antagonistes.

RÉSULTATS DU TEST DE BERENBAUM

Tableau 5 : Doses d'actifs nécessaires pour obtenir une prolifération bactérienne de 50%

| FORMULES | *S aureus* | *P aeruginosa* |
|---|---|---|
| Formule 2 | $A_{éq} = 6.10^{-3}$ % | $A_{éq} = 3.10^{-3}$ % |
| Formule 3 | $B_{éq} = 9.10^{-2}$ % | $B_{éq} = 9.10^{-2}$ % |
| Formule 5 (invention) | $A = 3.10^{-3}$ % | $A = 1,5.10^{-3}$ % |
| | $B = 3.10^{-2}$ % | $B = 1,5.10^{-2}$ % |
| **Indice de Berenbaum** | **0,8** | **0,7** |

L'indice de Berenbaum est inférieur à 1 pour les 2 souches bactériennes. La formule selon l'invention montre donc une action synergique des composés actifs.

**2. Comparaison de l'efficacité antibactérienne avec du miel ayant une activité peroxyde**

Des essais ont été réalisés avec du miel présentant une activité peroxyde équivalente (activité peroxyde de 0,025µg/g de miel par heure) auquel a été ajouté de l'oxyde de zinc, avec ou sans glucose oxydase.

Le protocole opératoire est le même qu'au point 1 a).

Les résultats obtenus sur la prolifération de *Pseudomonas aeruginosa* en présence de différentes compositions de miel diluées à une concentration de 6%, sont présentés dans le tableau 5bis ci-dessous :

Tableau 5bis : Importance de la présence de GOD pour obtenir un effet sur la prolifération bactérienne

| GOD | ZnO | % prolifération bactérienne |
|---|---|---|
| 0 U/g | 0% | 126% |

(suite)

| GOD | ZnO | % prolifération bactérienne |
|---|---|---|
| 0 U/g | 0,5% | 85% |
| 0 U/g | 1% | 58% |
| 0 U/g | 2% | 62% |
| 0,5 U/g | 1% | 0% |
| 5 U/g | 1% | 0% |
| 0,5 U/g | 2% | 0% |
| 5 U/g | 2% | 0% |

On constate que la seule activité peroxyde du miel ne suffit pas à obtenir l'activité antibactérienne et que la présence de glucose oxydase est nécessaire pour obtenir un effet synergique.

## 3. Tests de cytotoxicité

Afin d'évaluer la cytotoxicité des formules, un test de dénombrement cellulaire en présence de miel et d'une composition selon l'invention (formule 6) a été effectué sur des fibroblastes.

Protocole

Les produits sont dilués pour obtenir une concentration de 5,12% v/v de l'échantillon.
Le mode opératoire est le suivant :
Les cultures primaires de fibroblastes humains PAF 08052 sont ensemencées dans des microplaques 48 puits à raison de 10 000 cellules par puits avec un volume de milieu de culture complet de 500 µL. Les cellules vont ensuite adhérer au fond des puits durant 24 heures. Le milieu sera ensuite remplacé par le produit à tester dilué dans du milieu de culture privé de sérum. Le produit est testé à raison de 500 µL par puits et incubée pendant 48 heures à 37°C + 5% CO2. Les cellules sont ensuite décollées avec de la trypsine puis comptées sur lame de Malassez avec un colorant d'exclusion (bleu Trypan). Le pourcentage de prolifération fibroblastique est déterminé par rapport à un témoin non traité avec la composition.

Résultats

Les résultats sont présentés dans le tableau 6.

*Tableau 6: Dénombrement cellulaire sur fibroblastes en présence de formules à base de miel. Le témoin milieu correspond au milieu de culture seul ayant servi à diluer les échantillons (dilution en % v/v). Les cellules ont été traitées pendant 48h*

| Échantillons | Témoin milieu | Miel | Formule 6 |
|---|---|---|---|
| Pourcentage de prolifération fibroblastes à 1,28%(%) | 100% +/-21% | 100% +/-22% | 88% +/-22% |

La composition selon l'invention (formule 5) ne présente pas de cytotoxicité significative.

## 4. Efficacité in vivo

L'efficacité in vivo de la composition selon l'invention a été évaluée sur un panel de patients traités en structure de soin (maison de retraite, hôpitaux).
La formule 5 {Miel + GOD 0,1% + ZnO 1% + Excipients} a été testée sur 6 patients présentant des plaies en phase de détersion, fibrineuses (n=6) et/ou nécrosées (n=4), infectées (n= 1) ou non (n=5).
Les résultats de l'étude sont présentés dans le tableau 7 ci-dessous :

*Tableau 7*

| Type de plaie | Fibrine Oui/Non | Efficacité fibrine Mal/Assez bien/ Bien/Très bien | Colonisation bactérienne initiale Colonisation/ Infection/ absence de contamination | Efficacité sur colonisation Pas efficace/ Peu efficace/ Efficace/ très efficace/ Non concerné | Nécrose Oui/Non | Efficacité nécrose Pas efficace/ Peu efficace/ Efficace/ très efficace/ Non concerné | Inflammation Oui/Non | Efficacité inflammation Pas efficace/ Peu efficace/ Efficace/ très efficace/ Non concerné | Améliore la vitesse de cicatrisation Oui/Non | Appréciation comparative au miel seul Pas du tout efficace/ Moins efficace/ Equivalent/ Plus efficace |
|---|---|---|---|---|---|---|---|---|---|---|
| Ulcère mixte | Oui | Très bien | Absence de contamination | Non concerné | Non | | Non | Non concerné | Oui | Plus efficace |
| Escarre talonnière | Oui | Bien | Absence de contamination | Non concerné | Oui | Efficace | Non | Non concerné | Oui | Plus efficace |
| Escarre sacré | Oui | Bien | Absence de contamination | Non concerné | Oui | Très efficace | Non | Non concerné | Oui | Plus efficace |
| Escarre | Oui | Assez bien | Infection | Efficace | Oui | Efficace | Non | Efficace | Oui | Plus efficace |
| ulcère escarre | Oui | Bien | Absence de contamination | Non concerné | Oui | Efficace | Oui | Efficace | Oui | Plus efficace |
| ulcère, escarres | Oui | Très bien | Absence de contamination | Efficace | Non | | Oui | Efficace | Oui | Plus efficace |

EP 4 032 542 A1

On constate :

- 83% d'efficacité sur la fibrine

- Efficace sur plaie infectée

- 100% efficacité sur la nécrose

- 100% efficacité sur l'inflammation

- 100% d'efficacité sur l'accélération de la vitesse de cicatrisation

- 100% du panel testé considère que la formule testée est plus efficace pour la détersion par rapport au miel seul.

**Revendications**

1. Composition comprenant un mélange constitué par au moins un glucide, de la glucose oxydase et de l'oxyde de zinc, la glucose oxydase étant présente en une quantité d'au moins 0,025mU par gramme de composition.

2. Composition selon la revendication 1 **caractérisée en ce que** le mélange constitué d'au moins un glucide, de glucose oxydase et d'oxyde de zinc contient entre 1 et 99% de glucide en poids par rapport au poids total de la composition.

3. Composition selon l'une des précédentes revendications **caractérisée en ce que** le mélange constitué d'au moins un glucide, de glucose oxydase et d'oxyde de zinc contient entre 0,5 mU et 50 U de glucose oxydase par gramme de composition.

4. Composition selon l'une des précédentes revendications, **caractérisée en ce que** le mélange constitué d'au moins un glucide, de glucose oxydase et d'oxyde de zinc contient entre 0,1 et 40% d'oxyde de zinc en poids par rapport au poids total de la composition.

5. Composition selon l'une des précédentes revendications, **caractérisée en ce que** le au moins un glucide est choisi parmi le glucose, un miel d'origine naturelle choisi parmi miel de thym et/ou de miellat et/ou de sarrasin et/ou de manuka et/ou leurs mélanges, et du miel artificiel ou un mélange de miel(s) naturel(s) et artificiel(s).

6. Composition selon la revendication 5, **caractérisée en ce que** le miel artificiel est constitué majoritairement de glucose et/ou fructose et/ou saccharose.

7. Composition selon l'une des précédentes revendications, **caractérisée en ce qu'**elle comprend également au moins un constituant choisi parmi la vanilline, le caprylyl glycol, le pentylène glycol, le 1,2-hexanediol, un extrait de propolis, la vaseline, les paraffines, la lanoline, les pectines, l'amidon ou les dérivés d'amidon, les alginates ou les dérivés d'alginates, le chitosan ou les dérivés du chitosan, la cellulose ou les dérivés de celluloses, les gommes, les carraghénanes, les xanthanes, les béta-glucanes, , le méthylglyoxal, des polymères de synthèse, des cires, des huiles naturelles ou non, des beurres, des produits minéraux, des glycérides et autres esters gras, des tensio-actifs, l'eau, l'éthanol, le propylène glycol, les polyéthylènes glycols, le butylène glycol, le glycérol, le sorbitol, des composés de la matrice extracellulaire comme de l'acide hyaluronique ou du collagène, des hydrocarbures et silicones, des protéines et peptides.

8. Composition selon l'une des précédentes revendications, **caractérisée en ce qu'**elle se présente sous forme d'une poudre, sous forme liquide ou sous forme semi-liquide.

9. Composition selon l'une des précédentes revendications, **caractérisée en ce qu'**elle se présente sous forme de poudre à usage cutané, spray, gel, pommade, crème, émulsion, suppositoire ou ovule.

10. Composition selon l'une des précédentes revendications, **caractérisée en ce qu'**elle a été traitée par irradiation gamma.

**11.** Composition selon l'une des précédentes revendications, pour son utilisation comme produit antimicrobien à application topique.

**12.** Composition selon l'une des revendications 1 à 10, pour son utilisation comme produit de santé à application topique.

**13.** Composition selon l'une des revendications 1 à 10, pour son utilisation comme produit de santé anti-inflammatoire à application topique ou pour le traitement des irritations ou lésions cutanées.

**14.** Composition selon l'une des revendications 1 à 10, pour son utilisation comme produit de santé pour le traitement des brûlures, des désunions post-opératoires de cicatrice, des cavités résiduelles des sinus pilonidaux, des cicatrices chirurgicales infectées après mise à plat, des ulcères et escarres, des dermabrasions, des plaies traumatiques et/ou chirurgicales, des plaies chroniques, des plaies aigües, des plaies cancérologiques, des emplacements de stomies ou des plaies superficielles, des lésions de grattage, des érythèmes fessiers, de l'acné, des dermatites, des furoncles, des folliculites, des panaris, des mycoses ou de l'impétigo.

**15.** Composition selon l'une des revendications 1 à 10, pour son utilisation comme gel vaginal pour traiter des lésions cutanées et les infections microbiennes, comme crème rectale ou suppositoire pour traiter des lésions et irritations rectales, comme crème pour les mains et les pieds pour des crevasses et des engelures, baume pout la prévention et/ou le traitement des crevasses du mamelon, comme gel buccal pour traiter les aphtes et les mucites, ou comme stick ou baume à lèvre destiné à traiter les gerçures.

Europäisches
Patentamt

European
Patent Office

Office européen
des brevets

# RAPPORT DE RECHERCHE EUROPEENNE

Numéro de la demande

EP 22 15 6631

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 02/30467 A9 (TRITICUM EXPLOITATIE B V [NL]; POSTMES THEODORE JUSTIN LOUIS [NL] TRIT) 24 novembre 2011 (2011-11-24) * page 1, ligne 28 - page 2, ligne 1; revendications 1,3,6,8,10,12-14; exemple 2 * * page 4, lignes 2-7 * ----- | 1-15 | INV. A61K38/44 A61K31/7004 A61K33/30 A61K35/644 A61P17/02 A23L21/25 A61K9/00 |
| X | US 2001/006687 A1 (POSTMES THEO [NL]) 5 juillet 2001 (2001-07-05) * alinéa [0017] - alinéa [0018]; revendications 7-8,10 * ----- | 1-7 | A61K9/06 A61P17/00 A61P29/00 A61P31/02 A61P31/04 A61K31/702 |
| X | US 2008/233060 A1 (GRUNE GUERRY L [US]) 25 septembre 2008 (2008-09-25) * alinéa [0186]; revendications 4,9,16,17; exemples XXII,XXIV, XXXIV * ----- | 1-15 | |
| A | MOLAN P C: "Potential of honey in the treatment of wounds and burns", AMERICAN JOURNAL OF CLINICAL DERMATOLOGY, ADIS, US, vol. 2, no. 1, 1 février 2001 (2001-02-01) , pages 13-19, XP009168009, ISSN: 1175-0561 * le document en entier * ----- | 1-15 | **DOMAINES TECHNIQUES RECHERCHES (IPC)** A61K A61P |
| A | US 2009/012440 A1 (BRAY ROGER [GB] ET AL) 8 janvier 2009 (2009-01-08) * le document en entier * ----- | 1-15 | |
| A | GB 2 391 809 A (MEDIHONEY PTY LTD [AU]) 18 février 2004 (2004-02-18) * le document en entier * ----- | 1-15 | |
| A | FR 2 995 532 A1 (MELIPHARM [FR]) 21 mars 2014 (2014-03-21) * le document en entier * ----- | 1-15 | |

Le présent rapport a été établi pour toutes les revendications

| Lieu de la recherche | Date d'achèvement de la recherche | Examinateur |
|---|---|---|
| Munich | 15 juin 2022 | Kling, Isabelle |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...........................................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 22 15 6631

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-06-2022

| Document brevet cité au rapport de recherche | | | Date de publication | Membre(s) de la famille de brevet(s) | | | Date de publication |
|---|---|---|---|---|---|---|---|
| WO 0230467 | A9 | | 24-11-2011 | AT | 339224 | T | 15-10-2006 |
| | | | | AU | 1438902 | A | 22-04-2002 |
| | | | | AU | 2002214389 | A8 | 19-01-2012 |
| | | | | CA | 2425780 | A1 | 18-04-2002 |
| | | | | DE | 60123100 | T2 | 19-04-2007 |
| | | | | EP | 1331948 | A2 | 06-08-2003 |
| | | | | NL | 1016398 | C2 | 16-04-2002 |
| | | | | US | 2004131693 | A1 | 08-07-2004 |
| | | | | WO | 0230467 | A2 | 18-04-2002 |
| US 2001006687 | A1 | | 05-07-2001 | AT | 303727 | T | 15-09-2005 |
| | | | | AU | 3245601 | A | 09-07-2001 |
| | | | | BR | 0016650 | A | 24-12-2002 |
| | | | | CA | 2395328 | A1 | 05-07-2001 |
| | | | | EP | 1239742 | A1 | 18-09-2002 |
| | | | | MX | PA02006271 | A | 25-09-2003 |
| | | | | NL | 1013943 | C2 | 26-06-2001 |
| | | | | US | 2001006687 | A1 | 05-07-2001 |
| | | | | US | 2004170696 | A1 | 02-09-2004 |
| | | | | WO | 0147373 | A1 | 05-07-2001 |
| US 2008233060 | A1 | | 25-09-2008 | AUCUN | | | |
| US 2009012440 | A1 | | 08-01-2009 | AT | 439149 | T | 15-08-2009 |
| | | | | AU | 2002365662 | A1 | 17-06-2003 |
| | | | | CA | 2469033 | A1 | 12-06-2003 |
| | | | | EP | 1450871 | A1 | 01-09-2004 |
| | | | | ES | 2333112 | T3 | 17-02-2010 |
| | | | | GB | 2382527 | A | 04-06-2003 |
| | | | | JP | 2005511146 | A | 28-04-2005 |
| | | | | NZ | 545248 | A | 29-02-2008 |
| | | | | US | 2005033213 | A1 | 10-02-2005 |
| | | | | US | 2009012440 | A1 | 08-01-2009 |
| | | | | WO | 03047642 | A1 | 12-06-2003 |
| GB 2391809 | A | | 18-02-2004 | DE | 10337340 | A1 | 15-04-2004 |
| | | | | GB | 2391809 | A | 18-02-2004 |
| | | | | US | 2004121020 | A1 | 24-06-2004 |
| | | | | US | 2005255166 | A1 | 17-11-2005 |
| FR 2995532 | A1 | | 21-03-2014 | AUCUN | | | |

EPO FORM P0460

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2005112852 A
- WO 2007045931 A
- US 2004121020 A
- WO 2004000339 A
- FR 2995532
- FR 29995532
- FR 1258722

**Littérature non-brevet citée dans la description**

- **LANSDOWN ABG.** A pharmacological and toxicological profile of silver as an antimicrobial agent in medical devices. *Adv Pharm Sci 2010,* 24 Août 2010, 910686
- **BURD A ; KWOK CH ; HUNG SC, et al.** A comparative study of the cytotoxicity of silver-based dressings in monolayer cell, tissue explant, and animal models. *Wound Repair Regen,* 2007, vol. 15 (1), 94-104